# EUROPEAN PATENT APPLICATION

(11) **EP 4 524 113 A1**
(43) Date of publication of application: **19.03.2025**
(21) Application number: 24199936.6
(22) Date of filing: 12.09.2024
(51) Int. Cl.: C02F 11/18, C10G 1/00, C10G 1/06, C10L 9/08

(54) **METHOD OF CONVERTING A TEXTILE BASED FEEDSTOCK**

(30) Priority: 12.09.2023 DK PA202370469
(71) Applicant: Ecco Sko A/S, 6261 Bredebro (DK)
(72) Inventor: Lindhardt, Anders Thyboe, 6261 Bredebro (DK)
(74) Representative: Patentgruppen A/S

(57) **Abstract**

A method of converting a textile-based feedstock to a product is disclosed, the method comprising the steps of providing a textile-based feedstock, the textile-based feedstock having a moisture regain, wherein the textile-based feedstock has a moisture content above said moisture regain, adding an aqueous fraction to the textile-based feedstock to obtain a feedstock mixture, and subjecting the feedstock mixture to hydrothermal liquefaction. A system for converting a textile-based feedstock to a product is also disclosed.

## Description

### FIELD OF INVENTION

The invention relates to a method of converting a textile-based feedstock to one or more of biocrude, oligomeric components according to the claims. Also, the method relates to a system according to the claims.

### BACKGROUND

Currently, massive amounts of textiles are being discarded without being recycled even without recycling its components, whereby a big recycling potential is wasted.

One challenge is that waste textile fractions and recycle textiles fractions typically may be composed of a large number of different textile fiber types, which is a barrier for chemical conversion of the textile components. Furthermore, sorting of textiles by hand or automatically does not solve this problem, since it may be costly and since correct identification of textile types is not perfect. Also, textiles with mixed fiber types are also an issue.

An object of the present invention is to overcome these challenges.

### SUMMARY

The invention relates to a method of converting a textile-based feedstock to a product, the method comprising the steps of
providing a textile-based feedstock, the textile-based feedstock having a standard moisture regain, wherein the textile-based feedstock has a moisture content above said standard moisture regain,
adding an aqueous fraction to the textile-based feedstock to obtain a feedstock mixture, subjecting the feedstock mixture to hydrothermal liquefaction.

An advantage of the present invention may be that an efficient process may be obtained. In particular, by using wet textile-based feedstock, the feedstock may readily be subjected to hydrothermal liquefaction when the aqueous fraction has been added. In the present context, the term wet refers to the textile-based feedstock having a moisture content exceeding its standard moisture regain. In an embodiment of the invention, the wet textile-based feedstock may have a moisture content being at least 10 percentage points above its standard moisture regain, such as at least 20 percentage points above, such as at least 30 percentage points above, such as at least 40 percentage points above. In an embodiment of the invention, the textile-based feedstock as a whole, has a moisture content above said standard moisture regain of the textile-based feedstock as a whole.

A further advantage of the invention may be that the process may be more cost-efficient and faster, in particular with respect to preprocessing of the feedstock. For example, the method of the invention does not require drying of the feedstock but uses wet textile and textile-based feedstock. The reduced energy consumption facilitated by avoiding drying leads to a more cost-effective process and increasing the economic feasibility of the process.

A further advantage of the invention may be that any preprocessing such as shredding or processing into a particulate, of the textile-based feedstock prior to the hydrothermal liquefaction may be easier than for dry textile, since the water content of the textile may itself facilitate the preprocessing e.g. by acting as a lubricant or coolant during shredding. Also, using a wet textile-based feedstock may minimize dust formation and thus reduce a work hazard that may otherwise require increased safety precautions in the form of respirators. Additionally, reduction of dust may substantially reduce fire hazards.

A further advantage of the invention may be that due to the water content of the textile-based feedstock, additional water from the aqueous fraction does not need to overcome surface tension or capillary forces in the in order to fully mix with and wet the textile-based feedstock, or at least that such obstacles may be minimized. Thereby, any further processing such as mixing and soaking in order to obtain a reliably pumpable and adequately uniform feedstock mixture may be minimized and thereby processing time may be reduced.

A further advantage of the invention may be that any undesirable impurities, in particular inorganics such as sand, may be removed easier due to the wet state of the textile. Especially when using waste textile in the textile-based feedstock, it may be desirable to reduce the content of especially inorganic impurities such as sand and dust. Therefore, using wet textile in the presence of excess water may facilitate the partial or complete removal of this content. In contrast, the removal of sand and dust from completely dry textiles are typically inefficient and impractical. Resultingly, the method of the invention may use a wide range of textile-based feedstocks as input, including textile fractions that are unsuitable for recycling due to impurities, stains, and even partial decomposed textile from landfills, etc. In more detail, textile comprising e.g. food-based stains or other stains comprising organic material, such as e.g. paint, are processable by the present invention, since organic material are decomposable by hydrothermal liquefaction. Similarly, textile comprising undesirable impurities, such as mold, are processable by the present invention, as such impurities, including mold and undesirable mycotoxins therefrom, are decomposable by hydrothermal liquefaction.

Also, the process may advantageously minimize consumption of water by using the water content provided in the textile-based feedstock and thus lowering the need for addition of water as part of the aqueous fraction.

In the present context the term "textile-based feedstock", also sometimes simply referred to as "feedstock", is understood as a feedstock based on textile, i.e. containing mainly textile components with optional non-textile components. In an embodiment of the invention, the textile-based feedstock comprises textile in an amount of at least 50% by weight of dry matter, such as at least 60% by dry weight of dry matter, such as at least 70% by dry weight of dry matter, such as at least 80% by dry weight of dry matter. In some embodiments, the textile-based feedstock comprises non-organic components such as sand. In some embodiments, the textile-based feedstock comprises non-textile components originating from zippers (in particular metal or plastic components), buttons (in particular wooded or plastic components), other non-textile components (e.g. suede and/or leather), food, dirt (e.g. from ground contact in landfills etc.), or other organic and/or inorganic residues (e.g. from contact with other types of waste.

Thus, it is understood in the present context that the textile-based feedstock has a moisture content above the standard moisture regain of the textile-based feedstock. The moisture regain refers to the amount of moisture that textile-based feedstock is able to reabsorb after it has been dried. Specifically, according to an embodiment of the invention, the standard moisture regain refers to the amount of water resorbed by a dried material at equilibrium with standard atmosphere, i.e. conditions of temperature and humidity, compared to the mass of the dried material. In an embodiment of the invention, the standard moisture regain is understood in accordance with ASTM D2654-22. In an embodiment of the invention, the standard conditions are at 65% relative humidity at 20 degrees Celsius. In an embodiment of the invention, the standard conditions applied in accordance with ISO 139:2005. Thus, in an embodiment of the invention the textile-based feedstock is not pre-dried. It is also noted that in an embodiment of the invention, the textile-based feedstock thus may be considered wet in the sense that it has a higher content of water than a natural water content of the same textile-based feedstock at ambient conditions, such as at least 5% by weight higher water content, such as at least 10% by weight higher water content.

In an advantageous embodiment of the invention, the textile-based feedstock has a moisture content of at least 10 percentage points by weight above said standard moisture regain, such as at least 20 percentage points by weight above said standard moisture regain, such as at least 30 percentage points by weight above said standard moisture regain, such as at least 40 percentage points by weight above said standard moisture regain, such as at least 50 percentage points by weight above said standard moisture regain.

As an illustrative example, if the standard moisture regain of a certain example textile based feedstock is about 11% by weight and if the moisture content of the textile based feedstock is at least 10 percentage points above said standard moisture regain, this means that the moisture content is at least 21% by weight of the textile based feedstock.

In an embodiment of the invention, the textile-based feedstock has a moisture content of 10 to 90 percentage points by weight above said standard moisture regain, such as 20 to 80 percentage points by weight above said standard moisture regain, such as 30 to 70 percentage points by weight above said standard moisture regain, such as 40 to 60 percentage points by weight above said standard moisture regain, such as 50 to 60 percentage points by weight above said standard moisture regain.

In the present context, the term "textile" is understood as covering various fiber-based materials and fabrics, including woven and non-woven textile, knitted textile etc. Also, the fiber-based materials may be natural, synthetic or semi-synthetic in origin. Since both natural, synthetic and semi-synthetic materials are carbon-based or comprise carbon as a main constituent, the fibers are processable into a product of smaller components, such as components forming biocrude, and polymer components such as monomers and/or oligomers. It is noted that in the present context, the term "textile" includes both textiles used for apparel, furniture textiles, carpets, mats, shades, bedding textiles, and other consumer textile, textile from hotels and catering trade, as well as non-consumer textiles such as industrial textiles, geotextiles, medical textiles, agro-textiles, textiles used in vehicles, indutech, etc. It is noted that the above examples of textiles are not considered exhaustive but are merely given as non-limiting examples. Also, the above examples may in some cases be somewhat overlapping.

According to an advantageous embodiment of the invention, the textile-based feedstock comprises at least two different textile fibers, such as at least three different textile fibers, such as at least four different textile fibers.

According to an embodiment of the invention, the textile-based feedstock comprises a single type of different textile fibers.

According to an advantageous embodiment of the invention, the textile-based feedstock comprises cotton fibers.

According to an advantageous embodiment of the invention, the textile-based feedstock comprises polyethylene terephthalate fibers.

An advantage of the above embodiment may be the possibility of recovery of resulting terephthalic acid from the output of the hydrothermal liquefaction process, thereby providing a recycled version of terephthalic acid instead of its production relying directly on fossil sources.

According to an advantageous embodiment of the invention, the textile-based feedstock comprises cotton fibers and polyethylene terephthalate fibers.

According to an advantageous embodiment of the invention, the textile-based feedstock comprises polyurethane fibers.

According to an advantageous embodiment of the invention, the textile-based feedstock comprises acrylic fibers.

According to an advantageous embodiment of the invention, the textile-based feedstock comprises nylon fibers.

According to an advantageous embodiment of the invention, the textile-based feedstock comprises viscose fibers.

In an embodiment of the invention, the textile-based feedstock comprises cotton fibers, polyethylene terephthalate fibers, and acrylic fibers.

In an embodiment of the invention, the textile-based feedstock comprises cotton fibers, polyethylene terephthalate fibers, and nylon fibers.

In an embodiment of the invention, the textile-based feedstock comprises cotton fibers, polyethylene terephthalate fibers, and viscose fibers.

According to an advantageous embodiment of the invention, the textile-based feedstock comprises at least 50% by weight, such as at least 60% by weight, such as at least 70% by weight, such as at least 80% by weight, such as at least 90% by weight, of fibers selected from the group consisting of cotton fibers, polyethylene terephthalate fibers, polyurethane fibers, acrylic fibers, nylon fibers, modal fibers, viscose fibers, and any combination thereof,.

In an embodiment of the invention, the textile-based feedstock comprises 80 to 100% by weight of fibers selected from the group consisting of cotton fibers, polyethylene terephthalate fibers, polyurethane fibers, acrylic fibers, nylon fibers, modal fibers, viscose fibers, and any combination thereof, such as 80 to 99.9% by weight, such as 80 to 99% by weight.

According to an embodiment of the invention, the textile-based feedstock comprises textile fibers, wherein at least 80% by weight of textile fibers are selected from the group consisting of cotton fibers, polyethylene terephthalate fibers, polyurethane fibers, acrylic fibers, nylon fibers, modal fibers, viscose fibers, and any combination thereof.

According to an advantageous embodiment of the invention, the textile-based feedstock is selected from the group consisting of cotton fibers, polyethylene terephthalate fibers, polyurethane fibers, acrylic fibers, nylon fibers, modal fibers, viscose fibers, and any combination thereof.

According to an advantageous embodiment of the invention, the textile-based feedstock further comprises one or more fibers selected from the group consisting of silk fibers, wool fibers, linen fibers, and any combination thereof.

In an embodiment of the invention, the feedstock mixture further comprises non-textile apparel components.

In an embodiment of the invention, the feedstock mixture further comprises non-textile apparel components selected from the group consisting of suede, leather, wood, metal, non-textile polymeric components (e.g., plastics), and any combination thereof.

In an embodiment of the invention, the feedstock mixture further comprises non-textile apparel components selected from the group consisting of suede and/or leather.

In an embodiment of the invention, the textile-based feedstock comprises or consists of carpets.

According to an advantageous embodiment of the invention, the textile-based feedstock comprises textile in an amount of at least 50% by weight of the textile-based feedstock, such as at least 60% by weight of the textile-based feedstock, such as at least 70% by weight of the textile-based feedstock, such as at least 80% by weight of the textile-based feedstock, such as at least 90% by weight of the textile-based feedstock.

According to an advantageous embodiment of the invention, the textile-based feedstock has a water content of at least 10% by weight of the textile-based feedstock, such as at least 15% by weight of the textile based feedstock, such as at least 20% by weight of the textile based feedstock, such as at least 30% by weight of the textile based feedstock, such as at least 40% by weight of the textile based feedstock, such as at least 50% by weight of the textile based feedstock.

In an embodiment of the invention, the textile-based feedstock has a water content of 10 to 85% by weight of the textile-based feedstock, such as 15 to 80% by weight of the textile-based feedstock, such as 20 to 70% by weight of the textile-based feedstock, such as 30 to 60% by weight of the textile-based feedstock, such as 40 to 60% by weight of the textile-based feedstock, such as 50 to 60% by weight of the textile-based feedstock.

According to an advantageous embodiment of the invention, the feedstock mixture has a water content of at least 65% by weight of the feedstock mixture, such as at least 70% by weight of the feedstock mixture, such as at least 75% by weight of the feedstock mixture, such as at least 80% by weight of the feedstock mixture, such as at least 85% by weight of the feedstock mixture.

In an embodiment of the invention, the feedstock mixture has a water content of 65 to 99% by weight of the feedstock mixture, such as 70 to 98% by weight of the feedstock mixture, such as 75 to 95% by weight of the feedstock mixture, such as 80 to 95% by weight of the feedstock mixture, such as 85 to 90% by weight of the feedstock mixture.

According to an advantageous embodiment of the invention, the aqueous fraction comprises no more than 85% by weight of the feedstock mixture, such as no more than 80% by weight of the feedstock mixture, such as no more than 75% by weight of the feedstock mixture.

In an embodiment of the invention, the aqueous fraction comprises 50 to 85% by weight of the feedstock mixture, such as 60 to 80% by weight of the feedstock mixture, such as 70 to 75% by weight of the feedstock mixture.

In an embodiment of the invention, the feedstock mixture consists of the aqueous fraction, the textile-based feedstock, and additional components in an amount of no more than 10% by weight of the feedstock mixture, such as no more than 5% by weight of the feedstock mixture, such as no more than 2% by weight of the feedstock mixture, such as no more than 1% by weight of the feedstock mixture. In an embodiment of the invention, the feedstock mixture consists of the aqueous fraction and the textile-based feedstock.

According to an advantageous embodiment of the invention, the aqueous fraction comprises water in an amount of at least 80% by weight of the aqueous fraction, such as at least 90% by weight of the aqueous fraction, such as at least 95% by weight of the aqueous fraction, such as at least 99% by weight of the aqueous fraction.

In an embodiment of the invention, the aqueous fraction comprises water in an amount of 80 to 100% by weight of the aqueous fraction, such as 90 to 99.9% by weight of the aqueous fraction, such as 95 to 99.8% by weight of the aqueous fraction, such as 99 to 99.5% by weight of the aqueous fraction.

In an embodiment of the invention, the aqueous fraction consists essentially of water.

According to an advantageous embodiment of the invention, the feedstock mixture comprises at least 5% by weight of the textile-based feedstock, such as at least 10% by weight of the textile-based feedstock, such as at least 15% by weight of the textile-based feedstock, such as at least 20% by weight of the textile-based feedstock, such as at least 25% by weight of the textile-based feedstock.

In an embodiment of the invention, the feedstock mixture comprises 5 to 50% by weight of the textile-based feedstock, such as 10 to 45% by weight of the textile-based feedstock, such as 15 to 40% by weight of the textile-based feedstock, such as 20 to 35% by weight of the textile-based feedstock, such as 25 to 30% by weight of the textile-based feedstock.

According to an advantageous embodiment of the invention, the textile-based feedstock has an ash content of at least 0.2% by weight of the textile-based feedstock, such as at least 0.5% by weight of the textile-based feedstock, such as at least 1.0% by weight of the textile-based feedstock.

In an embodiment of the invention, the textile-based feedstock has an ash content of 0.2 to 5.0% by weight of the textile-based feedstock, such as 0.5 to 3.0% by weight of the textile-based feedstock, such as 1.0 to 2.0% by weight of the textile-based feedstock.

According to an advantageous embodiment of the invention, the textile-based feedstock comprises sand in an amount of at least 0.2% by weight of the textile-based feedstock, such as at least 0.5% by weight of the textile-based feedstock, such as at least 1.0% by weight of the textile-based feedstock.

In an embodiment of the invention, the textile-based feedstock comprises sand in an amount of 0.2 to 5.0% by weight of the textile-based feedstock, such as 0.5 to 3.0% by weight of the textile-based feedstock, such as 1.0 to 2.0% by weight of the textile-based feedstock.

According to an advantageous embodiment of the invention, the textile-based feedstock comprises inorganic components in an amount of at least 0.2% by weight of the textile-based feedstock, such as at least 0.5% by weight of the textile-based feedstock, such as at least 1.0% by weight of the textile-based feedstock.

In an embodiment of the invention, the textile-based feedstock comprises inorganic components in an amount of 0.2 to 5.0% by weight of the textile-based feedstock, such as 0.5 to 3.0% by weight of the textile-based feedstock, such as 1.0 to 2.0% by weight of the textile-based feedstock.

According to an advantageous embodiment of the invention, the textile-based feedstock comprises spores and/or fungi, such as mold, Cladosporium, Aspergillus, Penicillium, Alternaria, Fusarium, mildew, or any combination thereof.

An advantage of the above embodiment may be that the spores and/or fungi, or mycotoxins therefrom may be broken down by means of the hydrothermal liquefaction.

According to an embodiment of the invention, the textile-based feedstock comprises mold.

According to an embodiment of the invention, the textile-based feedstock comprises mycotoxin, such as ochratoxin A.

According to an embodiment of the invention, the textile-based feedstock comprises mycotoxin, such as ochratoxin A, in an amount of at least 1.0 microgram per kilogram of said textile-based feedstock.

According to an advantageous embodiment of the invention, the method further comprises a removal step of inorganic components, such as sand, of the textile-based feedstock prior to the step of hydrothermal liquefaction.

An advantage of the above embodiment may be that the textile-based feedstock may be used in hydrothermal liquefaction without costly pre-processing, such as e.g. drying. Thus, the sand or ash content may be lowered e.g. without removing any significant amount of water from the textile-based feedstock.

In an embodiment of the invention, the step of reducing inorganic components comprises mechanical removal of inorganic components, such as settling, such as gravity settling and/or centrifugal settling.

According to an advantageous embodiment of the invention, said product is selected from one or more of biocrude, oligomeric components, and monomeric components.

Thus, in the above embodiment, the method is a method for of converting the textile-based feedstock to one or more of biocrude, oligomeric components, and monomeric components.

It is noted that further outputs may be obtained from the method of the invention apart from said product. For example, an inorganic fraction, typically an inorganic solid fraction, may be obtained. Other outputs may also be obtained.

In an embodiment of the invention, said product comprises at least biocrude.

According to an advantageous embodiment of the invention, said product comprises at least monomeric components.

According to an advantageous embodiment of the invention, said product comprises at least biocrude and monomeric components.

In an embodiment of the invention, said product comprises at least oligomeric components.

In an embodiment of the invention, said product comprises at least oligomeric components and monomeric components.

According to an advantageous embodiment of the invention, the method further comprises a separation step after the hydrothermal liquefaction.

It is noted that in some embodiments, the separation step may comprise an extraction. Other physical and chemical separation processes may be used as well. For example, the separation step may comprise three-phase separation. Further examples include e.g. filtration and distillation.

According to an advantageous embodiment of the invention, the separation step comprises separating a monomeric component after the hydrothermal liquefaction.

It is noted in relation to the above embodiment that the monomeric component after separation may be part of a product fraction, which further comprises other components, such as oligomeric components, biocrude, etc.

In an embodiment of the invention, the separation step comprises separating an oligomeric component after the hydrothermal liquefaction.

It is noted in relation to the above embodiment that the oligomeric component after separation may be part of a product fraction, which further comprises other components, such as monomeric components, biocrude, etc.

According to an advantageous embodiment of the invention, the separation step comprises separating terephthalic acid after the hydrothermal liquefaction.

In an embodiment of the invention, the separation step comprises separating biocrude after the hydrothermal liquefaction.

It is noted in relation to the above embodiment that the biocrude after separation may be part of a product fraction, which further comprises other components, such as monomeric components, oligomeric components, etc.

According to an advantageous embodiment of the invention, the step of hydrothermal liquefaction is continuous.

Thus, in the above embodiment, at least the hydrothermal liquefaction is operated in continuous mode. Other steps may also be continuous, especially the step of adding the aqueous fraction, or may be batch-based.

In an embodiment of the invention, the method is continuous.

In an embodiment of the invention, the hydrothermal liquefaction is a batch process.

In an embodiment of the invention, the method is a batch process.

In an embodiment of the invention, the separation step is operated in a continuous mode.

According to an advantageous embodiment of the invention, the step of adding the aqueous fraction further comprises mixing the aqueous fraction with the textile-based feedstock to obtain the feedstock mixture.

According to an advantageous embodiment of the invention, the method further comprises a step of pre-processing the textile-based feedstock.

According to an advantageous embodiment of the invention, the pre-processing comprising shredding the textile-based feedstock to reduce the particle size.

According to an embodiment of the invention, the pre-processing comprising formulating the textile-based feedstock to a stable pumpable slurry used for continuous operation.

In an embodiment of the invention, the textile-based feedstock may be pre-processed externally and thus provided in a pre-processed form, e.g. as a stable pumpable slurry.

In an embodiment of the invention, the textile-based feedstock is pre-processed to an average particle size of no more than 10 cm, such as no more than 1 cm, such as no more than 5 mm.

In an embodiment of the invention, the textile-based feedstock is pre-processed to an average particle size of 0.01mm to 10 cm, such as 0.1mm to 1 cm, such as 1mm to 5 mm.

It is noted that in the context of the above embodiment, the term particle size refers to the longest dimension.

According to an advantageous embodiment of the invention, the step of hydrothermal liquefaction comprises subjecting the feedstock mixture to a temperature of at least 170 degrees Celsius, such as at least 200 degrees Celsius, such as at least 250 degrees Celsius, such as at least 300 degrees Celsius.

In an embodiment of the invention, the step of hydrothermal liquefaction comprises subjecting the feedstock mixture to a temperature of 170 to 550 degrees Celsius, such as 200 to 500 degrees Celsius, such as 250 to 450 degrees Celsius, such as 300 to 450 degrees Celsius.

According to an advantageous embodiment of the invention, the step of hydrothermal liquefaction comprises subjecting the feedstock mixture to a pressure of at least 50 bar, such as at least 75 bar, such as at least 100 bar, such as at least 125 bar.

In an embodiment of the invention, the step of hydrothermal liquefaction comprises subjecting the feedstock mixture to a pressure of least 50 to 350 bar, such as 75 to 300 bar, such as 100 to 250 bar, such as 125 to 200 bar.

According to an advantageous embodiment of the invention, the step of hydrothermal liquefaction has a processing time of at least 5 minutes, such as at least 10 minutes, such as at least 20 minutes, such as at least 30 minutes.

In an embodiment of the invention, the step of hydrothermal liquefaction has a processing time of 5 minutes to 10 hours, such as 10 minutes to 10 hours, such as 20 minutes to 5 hours, such as 30 minutes to 2 hours.

According to an advantageous embodiment of the invention, the method further comprises adding an additive, a reagent, a catalyst, or any combination thereof.

According to an embodiment of the invention, the method further comprises adding a catalyst to the feedstock mixture.

Adding a catalyst may advantageously facilitate increase of desired output components, such as biocrude and/or monomeric components.

The addition of catalyst may also serve to reduce the process temperature and/or process time needed to provide the desired output components. The addition of a catalyst may also serve to eliminate the formation of undesired process-impurities.

It is noted that in the present context, the term catalyst refers to additives having catalytic activity with respect to chemical reactions of the hydrothermal liquefaction step. It is noted that such catalyst may not necessarily be recoverable, e.g. due to inactivation by biproducts of certain other chemical processes. For example, base may be added as a catalyst for hydrolysis, but at the same time certain processes of the hydrothermal liquefaction may lead to formation of acids, which may hinder recovery of the bases. Similarly, recovery of catalysts may in some cases not be economically cost-effective. It is noted that in some cases, catalysts such as bases, may be referred to a catalytic additives, catalytic reagents, reaction-enhancing additives, or reaction-enhancing reagents.

In an embodiment of the invention, the catalyst is added to the feedstock mixture prior to the step of hydrothermal liquefaction.

According to advantageous an embodiment of the invention, the catalyst, the reagent, the additive or any combination therefore comprises a base.

According to an embodiment of the invention, the catalyst comprises a base.

In an embodiment of the invention, the base is selected from sodium hydroxide, potassium hydroxide, calcium hydroxide, calcium oxide, ammonium hydroxide, and any combination thereof.

In an embodiment of the invention, the catalyst comprises or consists of iron chloride, aluminum salts, a base, or any combination thereof.

In an embodiment of the invention, the catalyst comprises or consists of catalysts selected from the group consisting of transition metal catalysts, supported metal catalysts, noble metal catalysts, bifunctional catalysts, zeolites and heterogenous catalysts, biochar and biomass-derived catalysts, and any combination thereof.

According to an advantageous embodiment of the invention, the catalyst is provided as part of the aqueous fraction.

According to an embodiment of the invention, the additive, the reagent, the catalyst, or any combination thereof is provided as part of the aqueous fraction.

According to an advantageous embodiment of the invention, an aqueous output fraction is separated after the hydrothermal liquefaction and recirculated to form at least a part of the aqueous fraction.

The invention relates, in a further aspect, to a method of converting a textile-based feedstock to a product, the method comprising the steps of
providing a textile-based feedstock, wherein the textile-based feedstock has a moisture content of at least 10% by weight of the textile-based feedstock,
adding an aqueous fraction to the textile-based feedstock to obtain a feedstock mixture,
subjecting the feedstock mixture to hydrothermal liquefaction.

It is noted that this method may be combined with any embodiment of the invention, including the claims, with the provision that the limitations of claim 1 are not adhered to for the above described further aspect.

The invention further relates to a system for converting a textile-based feedstock to a product, the system comprising
a first inlet for receiving a textile-based feedstock having a standard moisture regain, wherein the textile-based feedstock has a moisture content above said standard moisture regain,
a second inlet for receiving an aqueous fraction,
a mixing zone arranged to receive the textile-based feedstock and the aqueous fraction and to output a feedstock mixture,
a hydrothermal liquefaction system arranged to receive the feedstock mixture.

According to an advantageous embodiment of the invention, the system according to the invention or any of its embodiments is configured to operate in accordance with the methods of the invention or any of its embodiments.

The invention further relates to a system for converting a textile-based feedstock to a product, the system comprising
a first inlet for receiving a textile-based feedstock having a moisture content of at least 10% by weight of the textile-based feedstock,
a second inlet for receiving an aqueous fraction,
a mixing zone arranged to receive the textile-based feedstock and the aqueous fraction and to output a feedstock mixture,
a hydrothermal liquefaction system arranged to receive the feedstock mixture.

According to an embodiment of the invention, the system described above is configured to operate in accordance with the methods of the invention or any of its embodiments.

The invention further relates to aspects and embodiments according to the following clauses.
1. A method of converting a textile-based feedstock (TBF) to a product, the method comprising the steps of
   providing a textile-based feedstock (TBF), the textile-based feedstock having a moisture regain, wherein the textile-based feedstock has a moisture content above said moisture regain,
   adding an aqueous fraction (AF) to the textile-based feedstock (TBF) to obtain a feedstock mixture (FM),
   subjecting the feedstock mixture (FM) to hydrothermal liquefaction (HTL).
2. The method according to clause 1, wherein the textile-based feedstock has a moisture content of at least 10 percentage points by weight above said standard moisture regain, such as at least 20 percentage points by weight above said standard moisture regain, such as at least 30 percentage points by weight above said standard moisture regain, such as at least 40 percentage points by weight above said standard moisture regain, such as at least 50 percentage points by weight above said standard moisture regain.
3. The method according to clause 1 or 2, wherein the textile-based feedstock (TBF) comprises at least two different textile fibers, such as at least three different textile fibers, such as at least four different textile fibers.
4. The method according to any of clauses 1-3, wherein the textile-based feedstock (TBF) comprises cotton fibers.
5. The method according to any of clauses 1-4, wherein the textile-based feedstock (TBF) comprises polyethylene terephthalate fibers.
6. The method according to any of clauses 1-5, wherein the textile-based feedstock (TBF) comprises cotton fibers and polyethylene terephthalate fibers.
7. The method according to any of clauses 1-6, wherein the textile-based feedstock (TBF) comprises polyurethane fibers.
8. The method according to any of clauses 1-7, wherein the textile-based feedstock (TBF) comprises acrylic fibers.
9. The method according to any of clauses 1-8, wherein the textile-based feedstock (TBF) comprises nylon fibers.
10. The method according to any of clauses 1-9, wherein the textile-based feedstock (TBF) comprises viscose fibers.
11. The method according to any of clauses 1-10, wherein the textile-based feedstock (TBF) comprises at least 80% by weight of fibers selected from the group consisting of cotton fibers, polyethylene terephthalate fibers, polyurethane fibers, acrylic fibers, nylon fibers, modal fibers, viscose fibers, and any combination thereof.
12. The method according to any of clauses 1-11, wherein the textile-based feedstock (TBF) is selected from the group consisting of cotton fibers, polyethylene terephthalate fibers, polyurethane fibers, acrylic fibers, nylon fibers, modal fibers, viscose fibers, and any combination thereof.
13. The method according to any of clauses 1-12, wherein the textile-based feedstock (TBF) further comprises one or more fibers selected from the group consisting of silk fibers, wool fibers, linen fibers, and any combination thereof.
14. The method according to any of clauses 1-13, wherein the textile-based feedstock comprises textile in an amount of at least 50% by weight of the textile-based feedstock, such as at least 60% by weight of the textile-based feedstock, such as at least 70% by weight of the textile-based feedstock, such as at least 80% by weight of the textile-based feedstock, such as at least 90% by weight of the textile-based feedstock.
15. The method according to any of clauses 1-14, wherein the textile-based feedstock (TBF) has a water content of at least 10% by weight of the textile-based feedstock, such as at least 15% by weight of the textile-based feedstock, such as at least 20% by weight of the textile based feedstock, such as at least 30% by weight of the textile based feedstock, such as at least 40% by weight of the textile based feedstock, such as at least 50% by weight of the textile based feedstock.
16. The method according to any of clauses 1-15, wherein the feedstock mixture (FM) has a water content of at least 65% by weight of the feedstock mixture, such as at least 70% by weight of the feedstock mixture, such as at least 75% by weight of the feedstock mixture, such as at least 80% by weight of the feedstock mixture, such as at least 85% by weight of the feedstock mixture.
17. The method according to any of clauses 1-16, wherein the aqueous fraction (AF) comprises no more than 85% by weight of the feedstock mixture (FM), such as no more than 80% by weight of the feedstock mixture (FM), such as no more than 75% by weight of the feedstock mixture (FM).
18. The method according to any of clauses 1-17, wherein the aqueous fraction (AF) comprises water in an amount of at least 80% by weight of the aqueous fraction (AF), such as at least 90% by weight of the aqueous fraction (AF), such as at least 95% by weight of the aqueous fraction (AF), such as at least 99% by weight of the aqueous fraction (AF).
19. The method according to any of clauses 1-18, wherein the feedstock mixture (FM) comprises at least 5% by weight of the textile-based feedstock, such as at least 10% by weight of the textile-based feedstock, such as at least 15% by weight of the textile-based feedstock, such as at least 20% by weight of the textile-based feedstock, such as at least 25% by weight of the textile-based feedstock.
20. The method according to any of clauses 1-19, wherein the textile-based feedstock (TBF) has an ash content of at least 0.2% by weight of the textile-based feedstock, such as at least 0.5% by weight of the textile-based feedstock, such as at least 1.0% by weight of the textile-based feedstock.
21. The method according to any of clauses 1-20, wherein the textile-based feedstock (TBF) comprises sand in an amount of at least 0.2% by weight of the textile-based feedstock, such as at least 0.5% by weight of the textile-based feedstock, such as at least 1.0% by weight of the textile-based feedstock.
22. The method according to any of clauses 1-21, wherein the textile-based feedstock (TBF) comprises inorganic components in an amount of at least 0.2% by weight of the textile-based feedstock, such as at least 0.5% by weight of the textile-based feedstock, such as at least 1.0% by weight of the textile-based feedstock.
23. The method according to any of clauses 1-22, wherein the textile-based feedstock (TBF) comprises spores and/or fungi, such as mold, Cladosporium, Aspergillus, Penicillium, Alternaria, Fusarium, mildew, or any combination thereof.
24. The method according to any of clauses 1-23, wherein the method further comprises a removal step of inorganic components, such as sand, of the textile-based feedstock prior to the step of hydrothermal liquefaction.
25. The method according to any of clauses 1-24, wherein said product is selected from one or more of biocrude, oligomeric components, and monomeric components.
26. The method according to any of clauses 1-25, wherein said product comprises at least monomeric components.
27. The method according to any of clauses 1-26, wherein said product comprises at least biocrude and monomeric components.
28. The method according to any of clauses 1-27, wherein the method further comprises a separation step after the hydrothermal liquefaction.
29. The method according to any of clauses 1-28, wherein the separation step comprises separating a monomeric component after the hydrothermal liquefaction.
30. The method according to any of clauses 1-29, wherein the separation step comprises separating terephthalic acid after the hydrothermal liquefaction.
31. The method according to any of clauses 1-30, wherein the step of hydrothermal liquefaction is continuous.
32. The method according to any of clauses 1-31, wherein the step of adding the aqueous fraction further comprises mixing the aqueous fraction with the textile-based feedstock to obtain the feedstock mixture.
33. The method according to any of clauses 1-32, wherein the method further comprises a step of pre-processing the textile-based feedstock.
34. The method according to any of clauses 1-33, wherein the pre-processing comprising shredding the textile-based feedstock to reduce the particle size.
35. The method according to any of clauses 1-34, wherein the step of hydrothermal liquefaction comprises subjecting the feedstock mixture to a temperature of at least 170 degrees Celsius, such as at least 200 degrees Celsius, such as at least 250 degrees Celsius, such as at least 300 degrees Celsius.
36. The method according to any of clauses 1-35, wherein the step of hydrothermal liquefaction comprises subjecting the feedstock mixture to a pressure of at least 50 bar, such as at least 75 bar, such as at least 100 bar, such as at least 125 bar.
37. The method according to any of clauses 1-36, wherein the step of hydrothermal liquefaction has a processing time of at least 5 minutes, such as at least 10 minutes, such as at least 20 minutes, such as at least 30 minutes.
38. The method according to any of clauses 1-37, wherein the method further comprises adding an additive, a reagent, a catalyst, or any combination thereof to the feedstock mixture.
39. The method according to any of clauses 1-38, wherein the additive, the reagent, the catalyst, or any combination thereof comprises a base.
40. The method according to any of clauses 1-39, wherein the catalyst is provided as part of the aqueous fraction.
41. The method according to any of clauses 1-40, wherein an aqueous output fraction is separated after the hydrothermal liquefaction and recirculated to form at least a part of the aqueous fraction.
42. A system (SYS) for converting a textile-based feedstock to a product, the system comprising
   a first inlet for receiving a textile-based feedstock (TBF) having a standard moisture regain, wherein the textile-based feedstock has a moisture content above said standard moisture regain,
   a second inlet for receiving an aqueous fraction (AF),
   a mixing zone (MZ) arranged to receive the textile-based feedstock (TBF) and the aqueous fraction (AF) and to output a feedstock mixture (FM),
   a hydrothermal liquefaction system arranged to receive the feedstock mixture (FM).
43. The system (SYS) according to clause 42, where the system (SYS) is configured to operate in accordance with the method of any of clauses 1-41.

### FIGURES

The invention will now be described with reference to the figures, where
Figure 1 illustrates a method according to an embodiment of the invention,
Figure 2 illustrates a method according to an embodiment of the invention, and
Figure 3 illustrates a system according to an embodiment of the invention.

### DETAILED DESCRIPTION

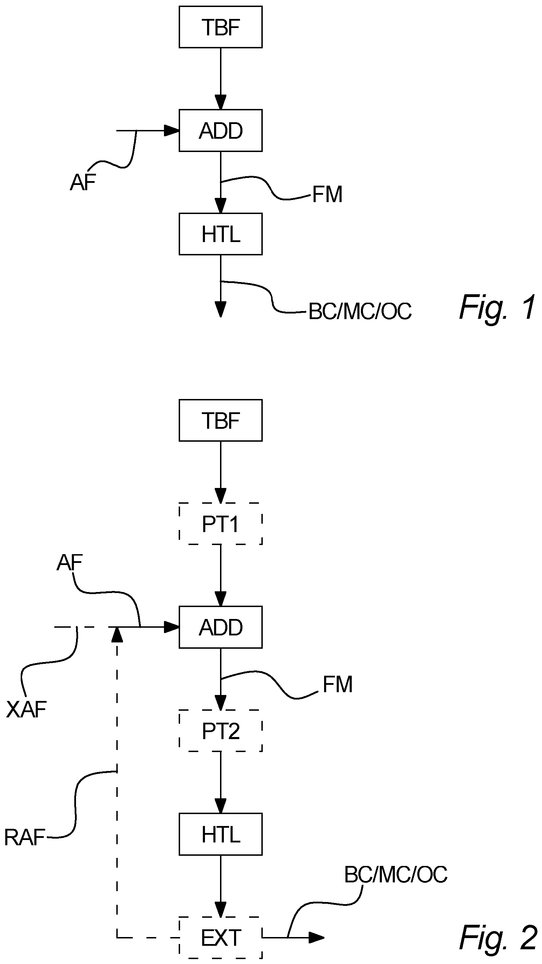

Referring to figure 1, a method for converting a textile-based feedstock TBF to one or more of biocrude BC, oligomeric components OC, and monomeric components MC is illustrated.

The method comprises first a step of providing a textile-based feedstock TBF having a standard moisture regain and where the textile-based feedstock TBF has a moisture content above said standard moisture regain,. Various textile fractions may be used, as long as the textile-based feedstock TBF has a moisture content above the standard moisture regain of the textile-based feedstock.

Then, an aqueous fraction AF is added to the textile-based feedstock TBF, whereby a feedstock mixture FM is obtained. This addition of the aqueous fraction AF optionally includes mixing in some embodiments. By adjusting the mixing ratio between the textile-based feedstock TBF and the aqueous fraction AF, the water content of the obtained feedstock mixture FM may be controlled.

The obtained feedstock mixture FM is then subjected to hydrothermal liquefaction HTL to obtain biocrude, oligomeric components, monomeric components, or a combination of these. The hydrothermal liquefaction may be continuous or may be batch process.

The hydrothermal liquefaction includes heating the feedstock mixture and maintaining it at a temperature above a certain predefined temperature. Since the feedstock mixture may be preheated in some embodiments, the need for further heating may vary between specific process embodiments. At the same time, the pressure is similarly kept above a certain value. Finally, the processing time or retention time may be adjusted to obtain a sufficient desirable degradation of the feedstock and at the same time avoid using too much energy for heating.

Now, referring to figure 2, a method for converting a textile-based feedstock to one or more of biocrude, oligomeric components, and monomeric components is illustrated.

It is noted that the method of figure 2 contains additional steps and elements compared to the method of figure 1, and that each of these additional steps or elements may in principle be applied separately within the context of the embodiment of figure 1, unless otherwise specified.

First, after providing the textile-based feedstock TBF, this may be subjected to a first pre-treatment PT1, which is illustrated as optional. The first pretreatment may include one or more of a separation (e.g. of inorganic elements, sand etc.), a shredding or processing into a particulate of the textile-based feedstock. Also, certain substances for enhancing the efficiency of the subsequent processing including the hydrothermal liquefaction may be added, e.g. a catalyst.

After adding the aqueous fraction AF, a second pre-treatment PT2 may be applied, which is illustrated as optional. It is noted that in the present context, the labeling of the "first" and "second" pre-treatment is in principle arbitrary and that each of these steps may be applied independently of whether the other pre-treatment step is applied.

The second pretreatment step PT2 may comprise the same and/or different processes as the first pretreatment step PT1 independently of whether the first pretreatment step PT 1 is applied or not. Thus, the second pretreatment step PT2 may include one or more of a separation (e.g. of inorganic elements, sand etc.), a shredding or processing into a particulate of the textile-based feedstock. Also, certain substances for enhancing the efficiency of the subsequent processing including the hydrothermal liquefaction may be added, e.g. an additive, a reagent, a catalyst, or any combination thereof.

Thus, the first and/or the second pretreatments step(s) PT1, PT2 may be configured to reduce inorganic components in the textile-based feedstock TBF directly or indirectly in the form of the feedstock mixture FM.

After the optional second pretreatment step PT2, the feedstock mixture FM is subjected to a hydrothermal liquefaction step HTL, which may be performed in accordance with the embodiment described in relation to figure 1.

The output of the hydrothermal liquefaction step HTL may be subjected to a separation step, which may separate one or more of monomeric components, oligomeric components, biocrude, and a reusable aqueous fraction RAF.

When recirculating the reusable aqueous fraction RAF, this may form the aqueous fraction AF added to the textile-based feedstock TBF to obtain the feedstock mixture FM, or it may be mixed with an external aqueous fraction XAF before adding to the textile-based feedstock TBF.

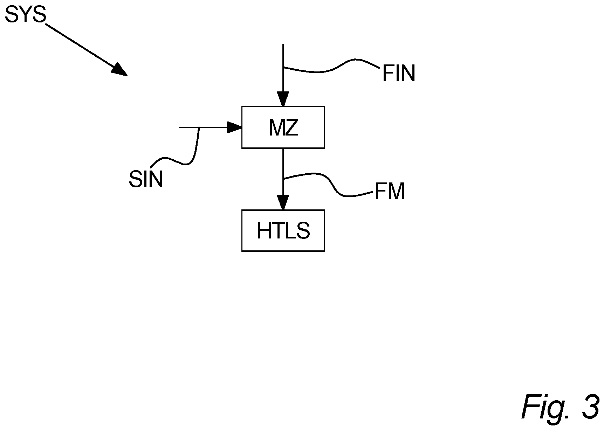

Now, referring to figure 3, a system SYS for converting a textile-based feedstock TBF to one or more of biocrude BC, and/or oligomeric components OC, and monomeric components MC is described in accordance with an embodiment of the invention. The system comprises a first inlet FI for receiving a textile-based feedstock TBF having a moisture content above said standard moisture regain,, a second inlet for receiving an aqueous fraction AF, a mixing zone MZ arranged to receive the textile-based feedstock TBF and the aqueous fraction AF and to output a feedstock mixture FM, and a hydrothermal liquefaction system HTLS arranged to receive the feedstock mixture FM.

## Claims

1. A method of converting a textile-based feedstock (TBF) to a product, the method comprising the steps of
providing a textile-based feedstock (TBF), the textile-based feedstock having a moisture regain, wherein the textile-based feedstock has a moisture content above said moisture regain,
adding an aqueous fraction (AF) to the textile-based feedstock (TBF) to obtain a feedstock mixture (FM),
subjecting the feedstock mixture (FM) to hydrothermal liquefaction (HTL).

2. The method according to claim 1, wherein the textile-based feedstock has a moisture content of at least 10 percentage points by weight above said standard moisture regain, such as at least 20 percentage points by weight above said standard moisture regain, such as at least 30 percentage points by weight above said standard moisture regain, such as at least 40 percentage points by weight above said standard moisture regain, such as at least 50 percentage points by weight above said standard moisture regain.

3. The method according to claim 1 or 2, wherein the textile-based feedstock (TBF) comprises at least two different textile fibers, such as at least three different textile fibers, such as at least four different textile fibers.

4. The method according to any of claims 1-3, wherein the textile-based feedstock (TBF) comprises cotton fibers.

5. The method according to any of claims 1-4, wherein the textile-based feedstock (TBF) comprises polyethylene terephthalate fibers.

6. The method according to any of claims 1-5, wherein the textile-based feedstock (TBF) comprises at least 80% by weight of fibers selected from the group consisting of cotton fibers, polyethylene terephthalate fibers, polyurethane fibers, acrylic fibers, nylon fibers, modal fibers, viscose fibers, and any combination thereof.

7. The method according to any of claims 1-6, wherein the textile-based feedstock comprises textile in an amount of at least 50% by weight of the textile-based feedstock, such as at least 60% by weight of the textile-based feedstock, such as at least 70% by weight of the textile-based feedstock, such as at least 80% by weight of the textile-based feedstock, such as at least 90% by weight of the textile-based feedstock.

8. The method according to any of claims 1-7, wherein the textile-based feedstock (TBF) has a water content of at least 10% by weight of the textile-based feedstock, such as at least 15% by weight of the textile-based feedstock, such as at least 20% by weight of the textile based feedstock, such as at least 30% by weight of the textile based feedstock, such as at least 40% by weight of the textile based feedstock, such as at least 50% by weight of the textile based feedstock.

9. The method according to any of claims 1-8, wherein the feedstock mixture (FM) comprises at least 5% by weight of the textile-based feedstock, such as at least 10% by weight of the textile-based feedstock, such as at least 15% by weight of the textile-based feedstock, such as at least 20% by weight of the textile-based feedstock, such as at least 25% by weight of the textile-based feedstock.

10. The method according to any of claims 1-9, wherein the textile-based feedstock (TBF) comprises inorganic components in an amount of at least 0.2% by weight of the textile-based feedstock, such as at least 0.5% by weight of the textile-based feedstock, such as at least 1.0% by weight of the textile-based feedstock.

11. The method according to any of claims 1-10, wherein said product is selected from one or more of biocrude, oligomeric components, and monomeric components.

12. The method according to any of claims 1-11, wherein the method further comprises a separation step after the hydrothermal liquefaction.

13. The method according to any of claims 1-12, wherein the separation step comprises separating a monomeric component after the hydrothermal liquefaction.

14. The method according to any of claims 1-13, wherein the step of hydrothermal liquefaction is continuous.

15. The method according to any of claims 1-14, wherein the pre-processing comprising shredding the textile-based feedstock to reduce the particle size.
